# EUROPÄISCHE PATENTSCHRIFT

(11) **EP 1 234 575 B1**
(45) Veröffentlichungstag und Bekanntmachung des Hinweises auf die Patenterteilung: **18.08.2004**
(21) Anmeldenummer: 01104398.1
(22) Anmeldetag: 23.02.2001
(51) Int. Cl.: A61K 31/34, C07D 307/46, A61P 35/00

(54) **Furanfettsäuren zur Steigerung der Effizienz der Zytostatikatherapie und/oder Bestrahlungstherapie**
Furan fatty acids for improving the efficiency of cytostatic and/or radiation therapy
Acides gras de furane pour l'augmentation de l'efficacité de la radiothérapie et/ou thérapie cytostatique

(43) Veröffentlichungstag der Anmeldung: 28.08.2002
(73) Patentinhaber: SIRS-Lab GmbH, 07745 Jena (DE)
(72) Erfinder: Deigner, Hans-Peter, Dr., 69514 Laudenbach (DE); Kinscherf, Ralf, Dr., 69198 Schriesheim (DE); Claus, Ralf A., Dr., 69190 Walldorf (DE); Russwurm, Stefan, Dr., 07743 Jena (DE)
(74) Vertreter: Schüssler, Andrea, Dr.

(56) Entgegenhaltungen:
- EP-A- 0 079 054
- US-A- 5 670 493
- FAWCETT ET AL.: "Natural acetylenes" J. CHEM. SOC., C, Bd. 19, 1968, Seiten 2455-2462, XP001007093

## Beschreibung

Die vorliegende Erfindung betrifft neue Furanfettsäuren, die sich dadurch auszeichnen, daß sie die Wirksamkeit einer Zytostatikatherapie und/oder Betrahlungstherapie erhöhen.

Sphingomyelin (*N*-acyl-sphingosyl-phosphoryl-cholin, SM) ist ein integraler Baustein sowohl biologischer Membranen als auch von Plasma-Lipoproteinen und eine wichtige Quelle zur Entstehung von Botenstoffen ("second messenger") wie Ceramid, das nach Stimulation durch Enzyme freigesetzt wird ("Sphingomyelin-pathway") Diese Signalübertragung durch Membransphingolipide wurde in den letzten Jahren intensiv untersucht. SM wird durch Isoformen eines der Phospholipase-C ähnlichen Enzyms, genannt Sphingomyelinase (SMase), metabolisiert. Bis heute sind fünf Isotypen der SMase bekannt. Die meisten Säugetierzellen sind in der Lage, Signale über den SM-Weg zu übertragen, wobei die Signalübertragung sowohl durch Rezeptoren wie zum Beispiel Tumor-Nekrose-Faktor (TNF)-Rezeptoren bzw. Interleukin-1-Rezeptoren als auch durch Streß wie z.B. UV-Licht, Oxidantien oder Strahlung aktiviert wird. Die Bildung von Ceramid wird heute als mit ausschlaggebend für die Einleitung lebenswichtiger Zellprozesse wie zum Beispiel Zellproliferation und Apoptose angesehen. Dieser Zusammenhang zwischen Ceramid-Bildung und Induktion der Apoptose beinhaltet eine Aktivierung jeweils bestimmter Isoformen der SMase, die in saurem (saure SMasen; acid SMases, aSMasen) bzw. in neutralem Milieu (neutrale SMasen; nSMasen) ihr pH-Optimum besitzen.

Apoptose, der programmierte Zelltod, verläuft in einer unumkehrbaren Sequenz morphologischer und biochemischer Veränderungen. Sie ist der wichtigste regulative Mechanismus zur Eliminierung nicht mehr benötigter Zellen während der embryonalen Entwicklung und des Wachstums oder irreparabel geschädigter Zellen. Verschiedene Noxen wie TNF/NGF, Hitzeschock, Zytostatika, ionisierende Strahlung, Infektionen durch Viren oder Bakterien, Entzug verschiedener Wachstumsfaktoren sowie zellmembranpermeable Ceramide induzieren Apoptose in unterschiedlichen Zelltypen. Ceramid hat als "second messenger" in verschiedensten Geweben Anteil am programmierten Zelltod. Es ist z.B. über eine Ceramid-aktivierte Protein-Kinase an der Regulierung der Signal-Transduktion beteiligt, was schließlich zu Apoptose führt.

Der Ceramid-induzierte Zelltod spielt möglicherweise eine Rolle bei verschiedensten Krankheiten wie: Sepsis, Atherosklerose, neurodegenerative Krankheiten wie z.B. Morbus Alzheimer, Morbus Parkinson und Infektionen durch Retroviren wie z.B. dem HIV-Virus, wobei jeweils von einer Ceramid-vermittelten Apoptose auszugehen ist. Der SM/Ceramid-Signalübertragungsweg ist auch an der Apoptose von Tumor-Zellen beteiligt. Reagenzien und Nährstoffe, die die Ceramid-Konzentration in Krebszellen erhöhen, sind somit theoretisch in der Lage, diese Zellen zu zerstören. Sie sind somit fähig, die Effizienz bekannter anti-Krebs-Medikamente zu erhöhen, wobei für einige Zytostatika die Bildung von Ceramid für ihre zytotoxischen Effekte sogar essentiell ist. Die Induktion des Zelltodes in den Krebszellen wird dabei dem angestiegenen zellulären Streß und der gestiegenen Ceramid-Konzentration zugeschrieben, was letztlich zur Apoptose führt. Es ist nämlich bekannt, daß bei der Krebstherapie durch Medikamente (z.B. Doxorubicin) oder radioaktive Bestrahlung, über eine Ceramidabhängige Zunahme der Expression des CD95/Apo-1/Fas-Rezeptor/Ligandensystem, eine Zunahme der Apoptoserate induziert wird (Herr et al., EMBO Journal 1997, 16(20), S. 6200-6208). Allerdings war es bisher nicht möglich, die Ceramid-Konzentration in Tumorzellen zu erhöhen, um dadurch die Effizienz bestimmter Zytostatika zu verbessern.

EP-A-0 079 054 beschreibt ein Mittel, das 9-β-D-arabinofuranosyladenin enthält und den antitumoralen Effeht anderer Mittel verstürkt.

Somit liegt der Erfindung im wesentlichen das technische Problem zugrunde, Mittel bereitzustellen, die die intrazelluläre Ceramid-Konzentration erhöhen, um damit die Wirksamkeit von Zytostatika zu steigern. Diese Mittel sollten aber selbst nicht zytotoxisch sein.

Die Lösung dieses technischen Problems wurde durch die Bereitstellung der in den Patentansprüchen gekennzeichneten Ausführungsformen erreicht.

Es wurde überraschenderweise gefunden, daß bei Vorinkubation von Tumorzellen mit bestimmten Furanfettsäure(FFS)-Verbindungen die nachfolgende Therapie mit bestimmten Zytostatika und/oder mit Bestrahlung zu einer starken Zunahme der Anzahl apoptotischer Zellen führt, wodurch die Wirkung der Zytostatika bzw. Bestrahlung eindeutig verbessert wird. Dabei führte vor allem die Verabreichung der nachfolgend definierten Verbindungen FFS **11a** und **11b** in Kombination mit Vinblastin oder Daunorubicin zu einer signifikanten Zunahme apoptotischer Zellen. Dieser Effekt nahm mit steigender FFS-Konzentration stark zu. Untersuchungen zum Mechanismus zeigten eine verstärkte Expression und Aktivität der aSMase, einhergehend mit einer Konzentrationserhöhung an intrazellulären Ceramid. Die kombinierte Behandlung mit den oben beschriebenen FFS Verbindungen führt somit zu einer Effizienzerhöhung der proapoptotischen Wirkung bestimmter Zytostatika, wie z. B. Vinblastin und Daunorubicin. Die therapeutische Anwendung der erfindungsgemäßen FFS Verbindungen zielt somit auf eine Optimierung und/oder Resistenzminderung/Verhinderung bei einer Zytostatikatherapie und/oder Bestrahlungstherapie ab. Die Möglichkeit der Verbesserung der Behandlungswirkung durch KombinationNorbehandlung mit den nicht-toxischen erfindungsgemäßen FFS Verbindungen erlaubt auch eine Dosisreduktion der Zytostatika bzw. Herabsetzung der Bestrahlungsdosis und damit auch eine Verbesserung der Verträglichkeit von Zytostatika und/oder Optimierung der Bestrahlungstherapie.

Somit betrifft die vorliegende Erfindung eine Verbindung mit der Formel 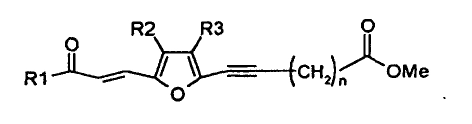 wobei
R1, R2 und R3, die gleich oder verschieden sein können, folgende Bedeutungen haben:
Wasserstoff,
Hydroxyl,
C₁ - C₂₀ Alkyl-, Alkenyl-, Alkinyl-, Alkylthiorest, O-Alkyl
COOH-, CONH₂-, CONH-Alkyl- , CON(Alkyl)₂ - Rest (mit Alkyl = C₁ - C₁₀)
Nitro,
Amino, Alkylaminorest (mit Alkyl = C₁ - C₁₀),
Aryl-, Aralkyl-, Alkoxyarylrest,
Halogen,
und
n eine ganze Zahl von 1-20, bevorzugt 1-14, ganz bevorzugt 6-10, weiter bevorzugt 8, ist, und
wobei die Verbindung außerdem dadurch gekennzeichnet ist, daß sie die Wirksamkeit einer Zytostatika- und/oder Bestrahlungstherapie steigern kann, oder
ein pharmazeutisch verträgliches Salz davon.

In der vorliegenden Erfindung bedeutet der Ausdruck "Alkyl-" eine geradlinige, verzweigte oder (poly)cyklische Alkylkette, die z.B. die Methyl-, Ethyl-, n-Propyl-, Isopropyl-, n-Butyl-, Isobutyl, sek-Butyl-, tert-Butyl-, n-Pentyl, Isopentyl-, Neopentyl-, tert-Pentyl-, 2-Methylbutyl-, n-Hexyl-, Isohexyl-, 2-Methylpentyl-, 2,3-Dimethylbutyl-, n-Heptyl-, 2-Methylhexyl-, 2,2-Dimethylpentyl-, 3,3-Dimethylpentyl-, 3-Ethylpentyl-, n-Octyl-, 2,2-Dimethylhexyl-, 3,3-Dimethylhexyl-, 3-Methyl-3-ethylpentyl-, Cyclopropyl-, Cyclobutyl-, Cyklopentyl-, Cylohexyl-, Cycloheptyl-, Cyclooctylgruppe umfaßt. Bevorzugt sind kurze Alkylketten, wie Methyl., Ethyl- oder Propyl-. Entsprechendes gilt für den O-Alkylrest.

In der vorliegenden Erfindung betrifft der Ausdruck "Alkenyl-" eine geradlinige, verzweigte oder (poly)cyclische Alkenylkette, die z.B. Vinyl-, Propenyl-, Isopropenyl-, Allyl-, 2-Methylallyl-, Butenyl-, Isobutenyl-, Pentenyl- oder Hexenyl- Isohexenyl-, Heptenyl-., Isoheptenyl-. Octenyl-, Isooctenylgruppen, Cyclobutenyl-, Cyclopentenyl-, Cyclohexenyl-, Cycloheptenyl-, Cyclooctenyl, Cyclononenyl- oder Cyclodecylgruppen umfaßt. Bevorzugt sind Vinyl-, Propenyl- und Isopropenyl-.

In der vorliegenden Erfindung betrifft der Ausdruck "Alkinyl-" eine geradlinige, verzweigte oder (poly)cyclische Alkinylkette, die z.B. Ethinyl-, Propinyl-, Isopropinyl-, Butinyl-, Isobutinyl-, Pentinyl-, Isopentinyl- oder Hexinylgruppen umfaßt.

Polycyclische Alkyl- bzw. Alkenylreste umfassen Norboman, Adamantan oder Benvalen.

In der vorliegenden Erfindung betrifft der Ausdruck "Aryl-" einen mono- oder polycyclischen C₆ - C₂₀ Arylrest. Beispiele hierfür sind ein carbocyclischer, monocyclischer Rest, beispielsweise die Phenylgruppe, ein heterocyclischer, monocyclischer Rest, beispielsweise die Gruppen Thienyl-, Furyl-, Pyranyl-, Pyrrolyl-, Imidazolyl-, Pyrazolyl-, Pyridyl- oder Pyrazinyl-. Diese Arylreste können unsubstituiert oder substituiert vorliegen. Geeignete Substituenten sind C₁ - C₄ Alkylgruppen (Definition s. vorstehend), Halogene, Nitro-, Hydroxy-, Carboxy-, Amino-, Carbamoyl-, C₂- C₆ Alkenyl- oder Alkinylgruppen (Definition s. vorstehend).

Der Begriff "Aralkyl-" umfaßt beispielsweise die Phenylethyl-, Phenylpropyl- Phenylbutyloder Phenylpentylgruppierung.

In der vorliegenden Erfindung umfaßt der Begriff "Halogen" Fluor, Chlor, Brom und lod, wobei Chlor und Brom bevorzugt sind.

Der Begriff "pharmazeutisch verträgliches Salz" umfaßt die Salze von Mineralsäuren, wie z.B. Hydrochlorid, Sulfat, Perchlorat, Nitrat und Phosphat, sowie die Salze organischer Säuren, wie z.B. Acetat, Oxalat, Succinat, Maleat und Fumarat.

Die erfindungsgemäßen Verbindungen können mit den in den nachfolgenden Beispielen 1 bis 15 beschriebenen Verfahren hergestellt werden. Ihre therapeutische Eignung, z.B. zur Verbesserung der Wirkung von Zytostatika und/oder Bestrahlungsbehandlung und die optimale Konzentration können mit den in dem nachstehenden Beispiel 16 beschriebenen Verfahren überprüft werden.

In einer bevorzugten Ausführungsform sind die Reste R1, R2 und R3 der erfindungsgemäßen Verbindung wie folgt:: R1 ist ein Methyl-, Ethyl- oder Propylrest und R2 und R3 ein Methylrest oder Wasserstoffatom sind.

Besonders bevorzugt ist eine Verbindung, bei der R1 ein Methylrest oder Ethylrest ist und R2 und R3 jeweils Wasserstoffatome sind.

Noch mehr bevorzugt ist eine Verbindung, bei der n 8 ist, wobei die am meisten bevorzugten Verbindungen 11-[5-(3-Oxo-1-butenyl)-furan-2-yl]-10-undecinsäuremethylester (11a) oder 11-[5-(3-Oxo-1-pentenyl)-furan-2-yl]-10-undecinsäuremethylester (11 b) sind.

Die vorliegende Erfindung betrifft auch Arzneimittel, die eine pharmakologisch wirksame Menge einer erfindungsgemäßen Verbindung zusammen mit einem pharmazeutisch verträglichen Träger enthalten. Geeignete Träger und die Formulierung derartiger Arzneimittel sind dem Fachmann bekannt. Zu geeigneten Trägem zählen beispielsweise Phosphat-gepufferte Kochsalzlösungen, Wasser, Emulsionen, beispielsweise Öl/Wasser-Emulsionen, Netzmittel, sterile Lösungen etc. Das erfindungsgemäße Arzneimittel kann in Form einer Injektionslösung, Tablette, Salbe, Suspension, Emulsion, eines Zäpfchens etc. vorliegen. Es kann auch in Form von Depots (Mikrokapseln, Zinksalze, Liposomen etc.) verabreicht werden. Die Art der Verabreichung des Arzneimittels hängt unter anderen davon ab, in welcher Form der Wirkstoff vorliegt, sie kann oral oder parenteral erfolgen. Zu den Verfahren für die parenterale Verabreichung gehören die topische, intra-arterielle (z.B. direkt zu einem Karzinom), intramuskuläre, intramedulläre, intrathekale, intraventrikuläre, intravenöse, intraperitoneale, transdermale oder transmukosale (nasal, vaginal, rektal, sublingual) Verabreichung. Die Verabreichung kann auch durch Mikroinjektion erfolgen. Die geeignete Dosierung wird von dem behandelnden Arzt bestimmt und hängt von verschiedenen Faktoren ab, beispielsweise von dem Alter, dem Geschlecht, dem Gewicht des Patienten, der Art und dem Stadium der Erkrankung, z.B. des Tumors, der Art der Verabreichung etc.

Die vorliegende Erfindung betrifft schließlich auch die Verwendung einer erfindungsgemäßen Verbindung zur Steigerung der Effizienz einer Zytostatikatherapie und/oder Bestrahlungstherapie oder zur Verhinderung oder Reduktion einer Resistenzbildung bei einer Zytostatikatherapie und/oder Bestrahlungstherapie, wobei vorzugsweise die Krebszellen vor der Therapiebeginn mit einer erfindungsgemäßen Verbindung vorbehandelt werden.

Die erfindungsgemäßen Verbindungen eignen sich prinzipiell in Kombination mit einer Bestrahlungsbehandlung. Außerdem können sie bei einer Zytostatikatherapie mit jedem Zytostatikum eingesetzt werden, wobei Zytostatika bevorzugt sind, für deren Wirkung die Bildung von Ceramid bzw. der Erhöhung der Ceramidkonzentration in den Krebszellen Voraussetzung ist. Beispiele für geeignete Zytostatika sind Vinblastin, Vincristin, Vinorelbin, Vindesin und andere Vinca-Alkaloide bzw. Analoge davon, Daunorubicin, Doxyrubicin, Aclarubicin, Epirubicin und andere Anthracykline bzw. Analoge davon, wobei Daunorubicin oder Vinblastin bevorzugt sind. Es ist auch möglich, daß weitere Zytostatika in Mehrfachkombinationen eingesetzt werden. Desweiteren kann vorher, gleichzeitig oder nachher eine Bestrahlungsbehandlung stattfinden.

### Legenden zu den Figuren

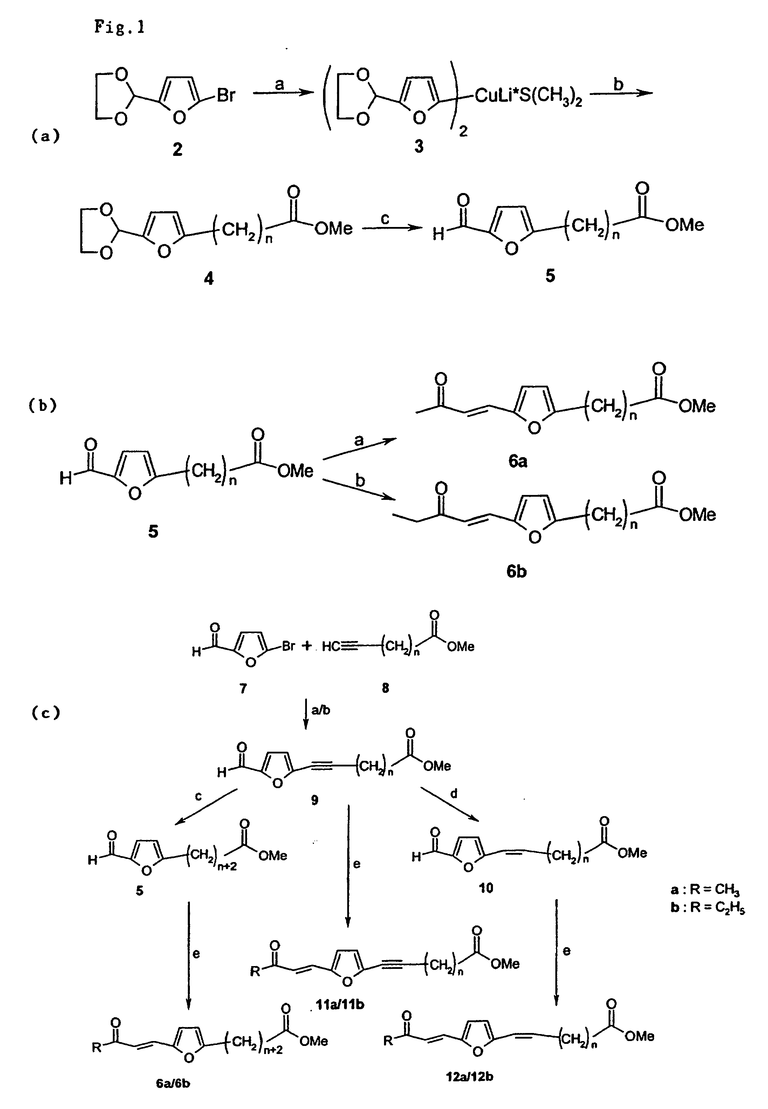

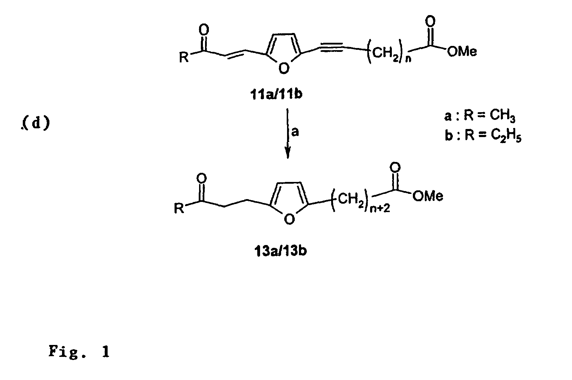

Figur 1: Schema der Synthese der Furanfettsäuren (FFS) über den Cuprat-Weg
   **(a)** a) Cul, S(CH₃)₂, n-BuLi, THF, -78°C, 1h; b) Br(CH₂)ₙCOOCH₃, THF, -40-0°C, 12h, 73%; c) 5%ige AcOH, Aceton, H₂O, RT, 2h, 88%.
   **(b)** *Wittig*-Reaktion: a) Ph₃PCHCOCH₃, Toluol, 110°C, 4h, 88%; b) Ph₃PCHCOC₂H₅, Toluol, 110°C, 4h, 76%.
   **(c)** Synthese der FFS. a) PdCl₂(PPh₃)₂, Cul, *i*-Pr₂NH, THF, RT, 24h, 81%; b) PdCl₂(PPh₃)₂, Cul, *i*-Pr₂NH, THF, 60°C, 2h, 82%; c) Lindlar-Kat., MeOH, H₂, 1 atm, 24h, 96%; d) Ruthenium auf Aluminium, MeOH, H₂, 1 atm, 36h, 92%; e) Ph₃PCHCOCH₃ bzw. Ph₃PCHCOC₂H₅, Toluol, 110°C, 4h.
   **(d)** Synthese der vollständig hydrierten FFS. a) Ruthenium auf Aluminium, MeOH, H₂, 1 atm, 36h, 68% bzw. 77%.
Figur 2: Natürlich vorkommende FFS **(14)**
Figur 3: Anzahl apoptotischer Renca-Zellen in Abhängigkeit von der FFS Konzentration Normiert auf die Zahl bei Inkubation mit 1 µM Daunorubicin ohne FFS.

Die nachstehenden Beispiele erläutern die Erfindung.

### Beispiel 1

### Allgemeine Synthese der Furanfettsäuren (FFS) über den Cuprat-Weg

Halogenierte Furane lassen sich leicht mit Halogenalkanen über reaktive Cuprate kuppeln¹. Zu diesem Zweck wurde acetalisiertes 5-Bromfurfural² **2** in das entsprechende Cuprat **3** überführt und anschließend mit einem terminalen Bromalkansäuremethylester Br(CH₂)ₙCOOCH₃ zu dem geschützten Furfuralderivat **4** gekuppelt (Figur 1a).

Die Entschützung des Acetals³ **4** zum Aldehyd **5** erforderte äußerst milde Bedingungen. Bei zu geringem pH-Wert sanken die Ausbeuten bis auf wenige Prozent ab, so daß die besten Ergebnisse mit 5%iger Essigsäure in einer Wasser-Aceton-Mischung zu erreichen waren. Der so erhaltene Aldehyd **5** wurde dann in einer *Wittig*-Reaktion⁴ zu den FFS **6a** und **6b** umgesetzt (Figur 1b).

Die Synthese der 3,4-unsubstituierten FFS gelang ausgehend von einem alkin-substituierten Furfural **9,** das durch Kupplung von 5-Brom-Furfural einem terminalen Alkinsäuremethylester nach *Sonogashira* unter basischen Bedingungen erhalten werden konnte. Aus diesem alkin-substituierten Furfural **9** ließen sich durch Hydrierung die entsprechenden alken- und alkansubstituierten Derivate **10** und **5** erhalten. Durch eine anschließende *Wittig*-Reaktion konnten die FFS mit verschiedener Kettenlänge und verschiedenem Hydrierungsgrad hergestellt werden (Figur 1c).

Die vollständig hydrierten Furanfettsäuren **13a** und **13b** wurden unter Verwendung von Ruthenium auf Aluminium als Hydrierungskatalysator aus den alkin-substituierten Furanfettsäuren **11a** bzw. **11b** erhalten (Figur 1d).

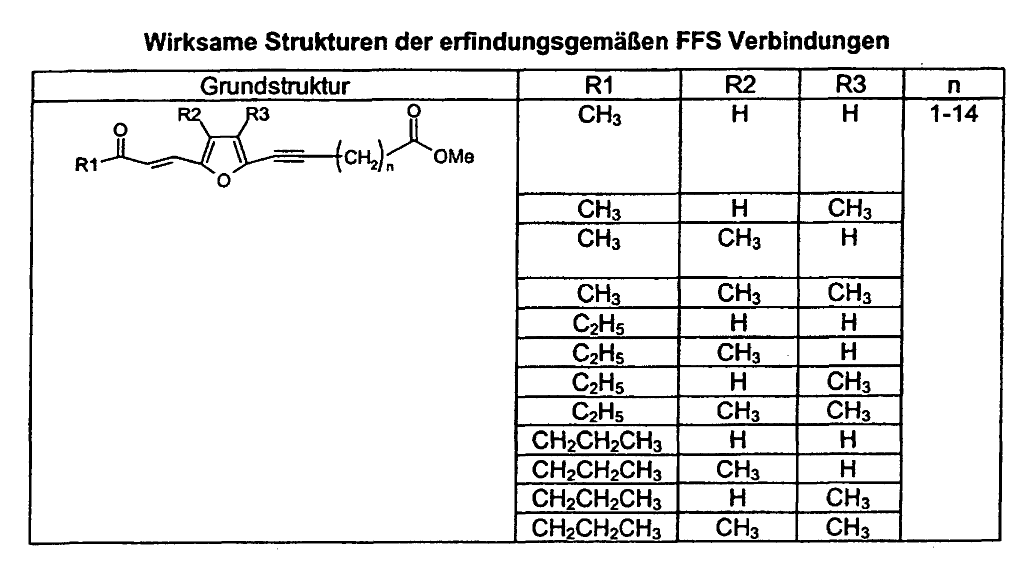

### Beispiel 2

### 11-(5-Formyl-furan-2-yl)-10-undecinsäure-methylester (9)

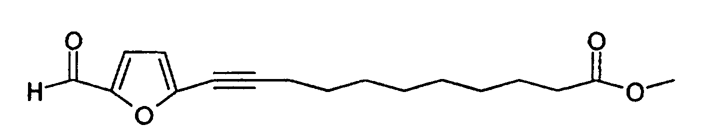

Es wurden 696 mg 5-Bromfurfural (4.0 mmol) in 30 ml trockenem THF gelöst. Dazu wurden im Argon-Gegenstrom 31 mg PdCl₂(PPh₃)₂ und 10 ml trockenes Diisopropylamin gegeben. Nach dreiminütigem Rühren wurden 16.5 mg Kupfer(I)iodid hinzugefügt, dabei wurde eine Verdunkelung beobachtet. Die Lösung wurde unter Rühren während 15 Minuten auf 60°C erhitzt. Dazu wurden dann 850 mg 10-Undecinsäuremethylester (4.34 mmol) in 10 ml trockenem THF gegeben, wobei sich nach ca. 15 Minuten ein flockiger Niederschlag bildete. Schließlich wurde für 2 Stunden unter Rückfluß erhitzt. Nach dem Abkühlen wurde mit 70 ml gesättigter Ammoniumchloridlösung hydrolysiert und drei mal mit je 50 ml Ethylacetat extrahiert. Die vereinigten organischen Phasen wurden über Magnesiumsulfat getrocknet und danach einrotiert. Nach säulenchromatographischer Reinigung (Aluminiumoxid // Ether) wurden 948 mg des Produkts in Form eines braunen Öls erhalten (Ausbeute: 9,48 g = 82%; R_{f}-Wert = 0.88).

^{**1**}**H-NMR** (CO(CD₃)₂): δ in ppm = 1.36 - 2.56 (m, 16H; 8CH₂); 3.61 (s, 3H; OCH₃); 6.77 (d, 1H, ³J = 3.44 Hz; C*H*_{*arom*}); 7.52 (d, 1 H, ³J = 3.45 Hz; C*H*_{*arom*}); 9.60 (s, 1H; CHO). ¹³C-**NMR** (CO(CD₃)₂): δ in ppm = 19.72/25.58/28.75/29.19/29.43/29.79/30.42/34.31 (CH2); 51.20 (O*C*H₃); 71.06 (C*C*Cₐᵣₒₘ); 99.28 (*C*CCₐᵣₒₘ); 116.94 (*C*Hₐᵣₒₘ); 122.87 (*C*Hₐᵣₒₘ); 142.70 (CCₐᵣₒₘ); 153.21 (CCₐᵣₒₘ); 174.74 (*C*OOCH₃); 177.74 (CHO). **MS** (EI, 89°C), m/z (%) = 290 (51.87) [M⁺]; 259 (37.15) [M⁺- OCH₃]; 230 (14.63) [M⁺- COOCH₃]; 175 (33.50) [C₁₁H₁₁O₂⁺]; 161 (38.01) [C₁₀H₉O₂⁺]; 147 (38.71) [C₉H₇O₂⁺]; 136 (77.07); 133 (50.61) [C₈H₇O₂⁺]; 59 (52.48) (COOCH₃⁺]; 55 (100.00). **IR** (Film): 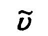 = 3344 (s); 3145 (s); 2932 (s); 2856 (s); 2227 (s); 1737 (s); 1681 (s); 1572 (w); 1501 (s); 1461 (m); 1389 (m); 1350 (m); 1261 (s); 1195 (m); 1098 (m); 1020 (s); 967 (m); 800 (s); 755 (m) cm⁻¹.
**HRMS** (C₁₇H₂₂O₄)[m/z]: 290.1518 (ber. 290.1518).

### Beispiel 3

### 11-(5-Formyl-furan-2-yl)-90-undecensäure-methylester (10)

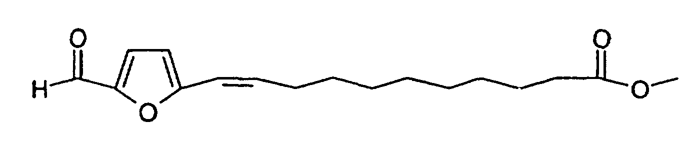

Eine Suspension von 1.45 g **9** (5 mmol) und 75 mg Lindlar-Katalysator in 30 ml trockenem Methanol wurde für 24 Stunden hydriert. Der Katalysator wurde abfiltriert und die Lösung einrotiert. Nach säulenchromatographischer Reinigung (Aluminiumoxid // n-Hexan : EE = 3 : 2) wurde das Produkt als hellbraunes Öl erhalten (Ausbeute: 1.40 g = 96%; R_{f}-Wert = 0.84).

^{**1**}**H-NMR** (CO(CD₃)₂): δ in ppm = 1.18 - 2.85 (m, 16H; 8CH₂); 3.61 (s, 3H; OCH₃); 5.93 (d, 1H, ³J= 11.82 Hz; CHCH(CH₂)₈); 6.32 (d, 1H, ³J= 11.76 Hz; C*H*CH(CH₂)₈); 6.63 (d, 1H, ³J = 3.69 Hz; CHₐᵣₒₘ ); 7.43 (d, 1H, ³J = 3.63 Hz; C*H*_{*arom*}); 9.61 (s,1H; CHO). ¹³C-**NMR** (CO(CD₃)₂): δ in ppm = 25.63 - 34.61 (8 CH₂); 51.43 (O*C*H₃); 112.61 (CH); 117.39 (*C*H); 124.12 (*C*Hₐᵣₒₘ); 138.45 (*C*Hₐᵣₒₘ); 152.55 (C*C*_{*arom*}); 158.92 (C*C*_{*arom*}); 174.10 (*C*OOCH₃); 177.70 (*C*HO).**MS** (EI, 89°C), m/z (%) = 292 (19.21) [M⁺]; 261 (69.86) [M⁺-OCH₃]; 149 (10.66) [C₉H₉O₂⁺]; 135 (20.73) [C₈H₈O₂⁺]; 122 (100.0) [C₇H₅O₂⁺]; 109 (20.76) [C₆H₄O₂⁺]; 79 (41.31) [C₅H₃O⁺]; 55 (41.73) [C₃H₃O⁺]; 41 (33.33) [C₂HO⁺]. **IR** (Film): 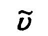 = 3343 (m); 3112 (s); 2932 (s); 2857 (s); 1740 (s); 1676 (s); 1571 (m); 1507 (m); 1465 (m); 1388 (m); 1350 (m); 1196 (m); 1021 (s); 967 (s); 803 (s); 766 (s) cm⁻¹. **HRMS** (C₁₇H₂₄O₄)[m/z]: 292.1677 (ber. 292.1676).

### Beispiel 4

### 11-(5-Formyl-furan-2-yl)-undecansäure-methylester (5)

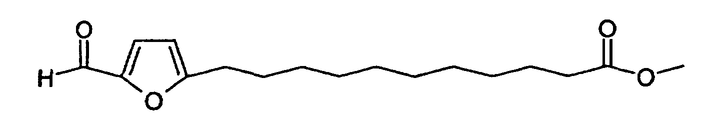

Eine Suspension von 1.45 g **9** (5 mmol) und 100 mg Ruthenium auf Aluminium in 20 ml trockenem Methanol wurde für 36 Stunden hydriert. Der Katalysator wurde abfiltriert und die Lösung einrotiert. Nach Säulenchromatographie (Aluminiumoxid // *n*-Hexan:EE = 3:2) wurde das Produkt als hellbraunes Öl erhalten (Ausbeute: 1.35 g = 92%; R_{f}-Wert = 0.90).

^{**1**}**H-NMR** (CO(CD₃)₂):δ in ppm = 1.21 - 1.72 (m, 16H; 8CH₂); 2.28 (t, 2H, ³J = 6.58 Hz; CH₂COOCH₃); 2.73 (t, 2H, ³J = 7.35 Hz; CH₂C₅H₃O); 3.62 (s, 3H; OCH₃); 6.13(d, 1H, ³J = 3.57 Hz; C*H*_{*arom*}); 7.34 (d, 1H, ³J = 3.60 Hz; *CH*_{*arom*}*);* 9.38(s, 1H; CHO). ^{**13**}**C-NMR** (CO(CD₃)₂): δ in ppm = 25.817 - 34.50 (10*C*H₂); 51.60 (O*C*H₃); 106.27 (*C*Hₐᵣₒₘ); 124.46 (C*H*_{*arom*}); 156.64 (C*C*_{*arom*}); 164.46 (C*C*_{*arom*}); 177.26 (COOCH₃); 177.54 (CHO). **MS** (El, 89°C). m/z (%) = 294 (17.18) [M⁺]; 279 (15.89) [M⁺- CH₃]; 253 ( 42.55) [M⁺- C₂HO₂]; 146 (12.54); 131 (14.81); 128 (26.85); 114 (16.88); 99 (33.76); 98 (11.25); 97 (11.75); 96 (23.20); 95 (28.63); 87 (13.72); 85 (30.60); 83 (22.90); 81 (22.31); 74 (16.88); 71 (100.0); 55 (59.23); 43 (59.03). **IR** (Film): 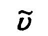 = 3466 (s); 3118 (s); 2927 (s); 2854 (s); 1739 (s); 1685 (s); 1581(w); 1517 (s); 1434 (m); 1260 (s); 1195 (m); 1171 (m); 1097 (m); 1019 (s); 797(s) cm⁻¹. **HRMS** (C₁₇H₂₆O₄)[m/z]: 294.1833 (ber. 294.1832).

### Beispiel 5

### 11-[5-(3-Oxo-1-butenyl) furan-2-yl]-10-undecinsäuremethylester (11a)

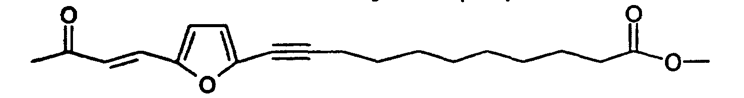

Eine Lösung von 1.08 g **9** (3.7 mmol) und 1.18 g 1-Triphenylphosphoranyliden-2-propanon (3.7 mmol) in 50 ml trockenem Toluol wurde für 4 Stunden unter Rückfluß gekocht. Nach dem Abkühlen wurde die Lösung eingeengt, in Dichlormethan aufgenommen und dreimal mit Wasser gewaschen. Nach abermaligem Einengen wurde der Rückstand in Diethylether aufgenommen und filtriert. Die Etherphase wurde getrocknet und konzentriert. Nach säulenchromatographischer Reinigung (Aluminiumoxid // n-Hexan : Essigester = 3 : 2 ) wurde das Produkt in Form eines braunen Öls erhalten (Ausbeute: 1.07 g = 88 %; R_{f}-Wert = 0.85).

^{**1**}**H-NMR** (CO(CD₃)₂): δ in ppm = 1.23 - 2.52 (m, 19H; 8 C*H*_{*2*}, 1 C*H*_{*3*}), 3.70 (s, 3H; OC*H*_{*3*}), 6.56 (d, 1H, ³*J* = 11.13 Hz; CHₐₗₖₑₙ), 6.66 (d, 1 H, ³*J* = 4.52 Hz; CHₐᵣₒₘ), 6.85 (d, 1H, ³*J* = 3.52 Hz; C*H*_{*arom*}), 7.31 (d, 1H, ³*J* = 7.06 Hz; *CH*_{*alken*})*,* ^{**13**}**C-NMR** (CO(CD₃)₂): δ in ppm = 19.77, 25.58, 28.91, 29.57, 29.59, 29.69, 30.56, 34.28 (8*C*H₂), 27.70 (*C*H₃), 51.41 (O*C*H₃), 71.62 (C*C*Cₐᵣₒₘ), 97.94 (*C*CCₐᵣₒₘ), 117.40 (*C*Hₐᵣₒₘ), 125.66 (*C*Hₐₗₖₑₙ), 128.97 (CHₐᵣₒₘ, CHₐₗₖₑₙ), 140.14 (CCₐᵣₒₘ), 151.76 (CCₐᵣₒₘ), 174.03 (*C*OOCH₃), 197.07 (*C*O). **MS** (EI, 124°C), m/z (%) = 330 (47.59) [M⁺], 315 (4.65) [M⁺-CH₃], 299 (5.90) [M⁺- OCH₃], 257 (8.18) [M⁺- CH₂COOCH₃], 201 (14.04) [M⁺- (CH₂)₅COOCH₃], 187 (28.38) [M⁺-(CH₂)₆COOCH₃], 173 (19.29) [M⁺- CH₂)₇COOCH₃], 159 (9.24) [C₁₀H₇O₂⁺]. 135 (30.24) [C₈H₇O₂⁺], 59 (15.86) [COOCH₃⁺], 43 (100.00) [H₃CCO⁺]. IR (Film): 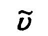 = 3458 (m), 3132 (s), 2932 (s), 2856 (s), 2224 (s), 1727 (s), 1667 (s), 1608 (s), 1562 (s), 1503 (s), 1438 (s), 1365 (s), 1260 (s), 1186 (s), 1099 (s), 1019 (s), 970 (s), 797 (s) cm⁻¹. **HRMS** (C₂₀H₂₆O₄)[m/z]: 330.1831 (ber. 330.1831).

### Beispiel 6

### 11-[5-(3-Oxo-1-butenyl)-furan-2-yl]-10-undecinsäure-methylester (11a)

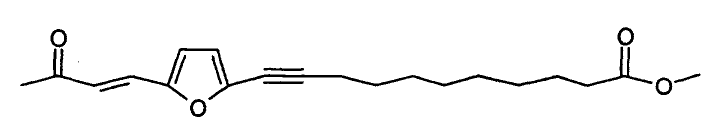

Es wurden 388 mg 1-(5-Bromo-2-furyl)-but-1-en-3-on (1.8 mmol) in 30 ml trockenem THF gelöst. Dazu wurden im Argon-Gegenstrom 12.6 mg PdCl₂(PPh₃)₂ und 5 ml trockenes Diisopropylamin gegeben. Dann wurde drei Minuten gerührt und es wurden 6.9 mg Kupfer(I)iodid hinzugefügt. Die Lösung wurde unter Rühren während 15 Minuten auf 60°C erhitzt. Dazu wurden 355 mg 10-Undecinsäuremethylester (1.8 mmol) in 10 ml trockenem THF gegeben. Nach ca. 15 Minuten bildete sich ein flockiger Niederschlag und es wurde für 18 Stunden unter Rückfluß erhitzt. Nach Abkühlen der Lösung wurde mit 70 ml gesättigter Ammoniumchloridlösung hydrolysiert und drei mal mit je 50 ml Essigester extrahiert. Die vereinigten organischen Phasen wurden über Magnesiumsulfat getrocknet und dann einrotiert. Nach säulenchromatographischer Reinigung (Aluminiumoxid // n-Hexan : Essigester = 3 : 2) wurde das Produkt in Form eines braunen Öls erhalten (Ausbeute: 550 mg = 84%; R_{f}-Wert = 0.85).

### Beispiel 7

### 11-[5-(3-Oxo-1-pentenyl)-furan-2-yl]-10-undecinsäure-methylester (11b)

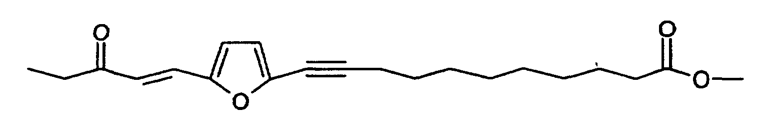

**11b** wurde analog wie **11a** durch Umsetzung von 1.08 g **9** (3.7 mmol) mit 1.23 g 1-Triphenylphosphoranyliden-2-butanon (3.7 mmol) hergestellt. Das Produkt wurde nach säulenchromatographischer Reinigung (Aluminiumoxid // n-Hexan : Essigester = 3 : 2) in Form eines braunen Öls erhalten (Ausbeute: 929 mg = 76%; R_{f}-Wert: 0.89).

^{**1**}**H-NMR** (CO(CD₃)₂): δ in ppm = 1.06 (t, 3H, ³*J* = 7.28 Hz; CH₃),1.21 - 2.52 (m, 16H; 8 CH₂), 2.68 (q, 2H, ³*J* = 7.31 Hz; CH₂CH₃), 3.62 (s, 3H; OCH₃), 6.62 (d, 1H, ³*J* = 3.47 Hz; CHₐₗₖₑₙ ), 6.68 (d, 1H, ³*J* = 4.57 Hz; C*H*_{*arom*}), 6.87 (d, 1H, ³*J* = 4.34 Hz; *CH*_{*arom*}*)* , 7.32 (d, 1H, ³*J* = 3.95 Hz; *CH*_{*alken*}*).* ^{**13**}**C-NMR** (CO(CD₃)₂):δ in ppm = 8.36 (CH₃), 19.70 - 34.61 (9*C*H₂), 51.43 (O*C*H₃), 71.79 (C*C*Cₐᵣₒₘ), 97.89 (*C*CCₐᵣₒₘ), 117.59 (*C*Hₐᵣₒₘ), 117.96 (*C*Hₐₗₖₑₙ), 124.71 (*C*Hₐₗₖₑₙ), 127.92 (*C*Hₐᵣₒₘ), 138.63 (C*C*_{*arom*}), 151.96 (C*C*_{*arom*}), 174.09 (*C*OOCH₃), 197.77 (*C*O). **MS** (El, 120°C), m/z (%) = 344 (78.61) [M⁺], 329 (8.83) [M⁺-CH₃], 315 (50.49) [M⁺- C₂H₅], 257 (22.43) [M⁺- (CH₂)₂COOCH₃], 215 (17.99) [M⁺-C₁₄H₂₅O₂], 201 (35.91) [M⁺- C₁₃H₂₃O₂], 187 (24.44) [M⁺- C₁₂H₂₁O₂], 149 (31.72) [C₉H₉O₂⁺], 57 (68.83) [C₃H₅O⁺], 55 (100.00) [C₃H₃O⁺]. **IR** (Film): 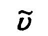 = 3114 (m), 2932 (s), 2856 (s), 2227 (m), 1733 (s), 1683 (s), 1506 (m), 1436 (m), 1366 (m), 1195 (m), 1020 (m), 968 (m), 802 (m) cm⁻¹. **HRMS** (C₂₁H₂₈O₄) [m/z]: 348.4817 (ber. 348.4814).

### Beispiel 8

### 11-[5-(3-Oxo-1-pentenyl)-furan-2-yl]-10-undecinsäuremethylester (11b)

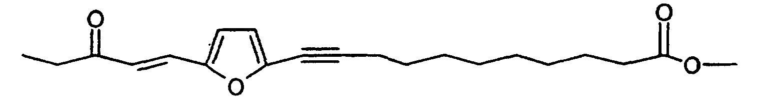

Es wurden 412 mg 1-(5-Bromo-2-furyl)-pent-1-en-3-on (1.8 mmol) in 30 ml trockenem THF gelöst. Im Argon-Gegenstrom wurden 12.6 mg PdCl₂(PPh₃)₂ und 5 ml trockenes Diisopropylamin zugefügt. Danach wurde drei Minuten gerührt und es wurden 6.9 mg Kupfer(I)iodid hinzugefügt. Die Lösung wurde unter Rühren während 15 Minuten auf 60°C erhitzt und eine Lösung von 355 mg 10-Undecinsäuremethylester (1.8 mmol) in 10 ml trockenem THF zugetropft. Nach ca. 15 Minuten bildete sich ein flockiger Niederschlag und es wurde für 18 Stunden unter Rückfluß erhitzt. Nach Abkühlen der Lösung wurde mit 70 ml gesättigter Ammoniumchloridlösung hydrolysiert und dreimal mit je 50 ml Essigester extrahiert. Die vereinigten organischen Phasen wurden über Magnesiumsulfat getrocknet und dann eingeengt. Nach säulenchromatographischer Reinigung (Aluminiumoxid // n-Hexan : Essigester = 3 : 2) wurde das Produkt in Form eines braunen Öls erhalten (Ausbeute: 489 mg = 78%; R_{f}-Wert = 0.89).

### Beispiel 9

### 11-[5-Oxo-1-butenyl)-furan-2-yl]-10-undecensäure-methylester (12a)

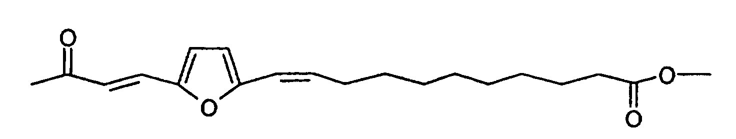

**12a** wurde analog zu **11a** aus 292 mg **10** (1 mmol) und 318 mg 1-Triphenylphosphoranyliden-2-propanon (1 mmol) hergestellt. **12a** wurde nach säulenchromatographischer Reinigung (Aluminiumoxid // n-Hexan : Essigester = 3:2) als braunes Öl erhalten (Ausbeute: 298 mg = 90%; R_{f}-Wert = 0.84).

^{**1**}**H-NMR** (CO(CD₃)₂): δ in ppm = 1.33-2.57 (m, 16H; 8 CH₂), 2.84 (s, 3H; *H*₃CCO), 3.60 (s, 3H; OCH₃), 5.78 (d, 1H, ³*J* = 11.78 Hz; C*H*_{*alken*}), 6.28 (d, 1H, ³*J* = 10.23 Hz; C*H*_{*alken*}), 6.51 (d, 1H, ³*J* = 10.39 Hz; CHₐₗₖₑₙ), 6.52 (d, 1H, ³*J* = 3.54 Hz; CHₐᵣₒₘ), 6.89 (d, 1H, ³*J* = 3.64 Hz; *CH*_{*arom*}*),* 7.38 (d, 1H, ³*J* = 16.02 Hz; *CH*_{*alken*} *).* ^{**13**}**C-NMR** (CO(CD₃)₂): δ in ppm = 25.65 - 34.32 (8CH₂), 51.43 (O*C*H₃), 113.20 (*C*Hₐᵣₒₘ), 118.07 (*C*Hₐₗₖₑₙ), 118.64 (*C*Hₐᵣₒₘ), 124.31 (*C*Hₐₗₖₑₙ), 129.75 (*C*Hₐₗₖₑₙ), 135.19 (*C*Hₐₗₖₑₙ), 150.85 (C*C*_{*arom*}), 156.58 (C*C*_{*arom*}), 174.10 (*C*OOCH₃), 197.16 (*C*O). **MS** (El, 113°C), m/z (%) = 332 (63.39) [M⁺], 317 (3.81) [M⁺- CH₃], 301 (9.04) [M⁺- OCH₃], 289 (8.54) [M⁺-CH₃CO], 189 (18.65) [C₁₂H₁₃O₂⁺], 175 (43.82) [C₁₁H₁₁O₂⁺], 162 (16.60) [C₁₀H₁₀O₂⁺], 147 (25.02) [C₉H₇O₂⁺], 135 (59.19) [C₈H₇O₂⁺], 108 (20.34) [C₇H₈O⁺], 95 (46.99) [C₆H₇O⁺], 59 (9.98) [COOCH₃⁺], 55 (24.00) [C₃H₃O⁺], 43 (100.0) [C₂H₃O⁺]. **IR** (Film): 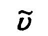 = 3446(s), 3027 (m), 2920 (s), 2849 (s), 1734 (s), 1682 (s), 1437 (m), 1364 (m), 1278 (m), 1186 (s), 1024(s), 969 (s), 887(w), 788 (s), 627 (w), 567 (w) cm⁻¹. **HRMS** (C₂₀H₂₈O₄)[m/z]: 332.1990 (ber. 332.1990).

### Beispiel 10

### 11-[5-Oxo-1-pentenyl)-furan-2-yl]-10-undecensäure-methylester (12b)

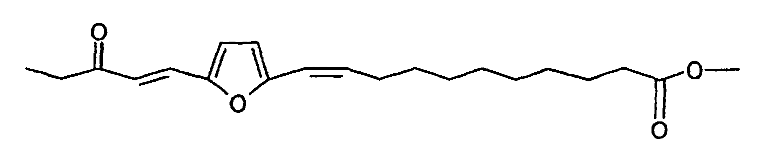

**12b** wurde analog zu **11a** aus 496 mg **10** (1.7 mmol) und 564 mg 1-Triphenylphosphoranyliden-2-butanon (1.7 mmol) hergestellt. **12b** wurde nach säulenchromatographischer Reinigung (Aluminiumoxid // n-Hexan : Essigester = 3 : 2) als braunes Öl erhalten (Ausbeute: 494 mg = 84%; R_{f}-Wert = 0.76).

^{**1**}**H-NMR** (CO(CD₃)₂): δ in ppm = 0.93 (t, 3H, ³*J* = 7.27 Hz; CH₂C*H*₃), 1.04 - 2.31 (m, 16H; 8 CH₂), 3.40 (q, 2H, ³*J* = 22.0 Hz; CH₂CH₃), 3.69 (s, 3H; OCH₃), 6.50 (d, 1H, ³*J* = 3.31 Hz; C*H*_{*arom*}), 6.58 (d, 1 H, ³*J* = 15.97 Hz; *CH*_{*alken*})*,* 6.62 (d, 1H, ³*J* = 15.43 Hz; *CH*_{*alken*})*,* 6.88 (d,1H, ³*J*= 3.40 Hz; CHₐᵣₒₘ) , 7.18 (d, 1H, ³*J*= 15.99 Hz; *CH*_{*alken*}*),* 7.33 (m, 1H; C*H*_{*alken*}). ^{**13**}**C-NMR** (CO(CD₃)₂): δ in ppm = 14.58 (*C*H₃), 25.67 - 34.65 (11*C*H₂), 51.41 (O*C*H₃), 110.29 (*C*Hₐᵣₒₘ), 118.42 (*C*Hₐᵣₒₘ), 123.36 (*C*Hₐₗₖₑₙ), 125.81 (*C*Hₐₗₖₑₙ), 128.63 (*C*Hₐₗₖₑₙ), 130.85 (*C*Hₐₗₖₑₙ), 152.44 (C*C*_{*arom*}), 155.08 (C*C*_{*arom*}), 174.11 (*C*OOCH₃), 199.28 (CO). **MS** (El, 121°C), m/z (%) = 346 (100.0) [M⁺], 317 (42.39) [M⁺- C₂H₅], 289 (16.36) [M⁺-C₂H₅CO], 259 (13.81) [M⁺- C₄H₇O₂], 149 (83.07) [C₉H₉O₂⁺], 147 (51.37) [C₉H₇O₂⁺], 109 (89.26) [C₇H₉O⁺], 57 (84.72) [C₃H₅O⁺], 55 (52.31) [C₃H₃O⁺]. **IR** (Film): 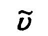 = 3121 (m), 2927 (s), 2855 (s), 1736 (s), 1682 (s), 1603 (s), 1437 (m), 1366 (m), 1254 (m), 1198 (s), 1174 (s), 1119 (s), 1021 (s), 971 (s), 788 (s), 721 (s) cm⁻¹.

### Beispiel 11

### 11-[5-Oxo-1-buteny-yl]-undecansäure-methylester (6a)

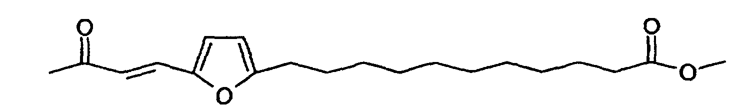

**6a** wurde analog zu **11a** aus 353 mg **5** (1.2 mmol) und 382 mg 1-Triphenylphosphoranyliden-2-propanon (1.2 mmol) dargestellt. **6a** wurde nach säulenchromatographischer Reinigung (Aluminiumoxid // n-Hexan : Essigester = 3:2) als hellbraunes Öl erhalten (Ausbeute: 293 mg = 73%; RᵣWert = 0.84).

^{**1**}**H-NMR** (CO(CD₃)₂):δ in ppm = 1.19 - 2.61 (m, 20H; 10 CH₂), 1.96 (s, 3H; CH₃CO), 3.28 (s, 3H; OCH₃), 5.28 (d, 1H, ³*J* = 15.35 Hz; C*H*_{*alken*}), 5.41 (d, 1H, ^{*3*}*J* = 15.40 Hz; *CH*_{*alken*}*),* 6.02 (d, 1 H, ^{*3*}*J* = 3.58 Hz; *CH*_{*arom*}*),* 6.29 (d, 1 H, ³*J* = 3.49 Hz; CHₐᵣₒₘ). ¹³C-**NMR** (CO(CD₃)₂): δ in ppm = 14.50 (CO*C*H₃), 25.65 - 34.63 (10*C*H₂), 51.41 (O*C*H₃), 105.99 (*C*Hₐᵣₒₘ), 109.67 (*C*Hₐₗₖₑₙ), 130.56 (*C*Hₐᵣₒₘ), 131.12 (CHₐₗₖₑₙ), 150.38 (CCₐᵣₒₘ), 157.01 (C*C*_{*arom*}), 174.06 (COOCH₃), 197.28 (CO). **MS** (EI, 96°C), m/z (%) = 334 (35.98) [M⁺], 303 (6.09) [M⁺- COCH₃], 149 (16.28) [C₉H₉O₂⁺], 135 (100.0) [C₈H₇O⁺], 95 (57.46) [C₆H₇O⁺], 81 (30.57) [C₅H₅O⁺], 55 (59.51) [C₃H₃O⁺], 43 (46.65) [C₂H₃O⁺]. **IR** (Film): 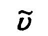 = 3447 (m), 3123 (m), 2926 (s), 2854 (s), 1739 (s), 1684 (s), 1612 (s), 1517 (m), 1456 (m), 1436 (m), 1362 (m), 1253 (m), 1192 (m), 1173 (m), 1110 (s), 1019 (m), 968 (m), 791 (m) cm⁻¹. **HRMS** (C₂₀H₃₀O₄)[m/z]: 334.4518 (ber. 334.4516).

### Beispiel 12

### 11-[5(3-Oxo-pentenyl)-furan-2-yl]-undecansäure-methylester (6b)

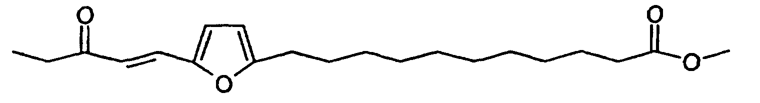

**6b** wurde analog zu **11a** aus 300 mg **5** (1.02 mmol) und 338 mg 1-Triphenylphosphoranyliden-2-butanon (1.02 mmol) dargestellt. **6b** wurde nach säulenchromatographischer Reinigung (Aluminiumoxid // n-Hexan : Essigester = 3:2) als braunes Öl erhalten (Ausbeute: 274 mg = 77%; RᵣWert = 0.90). ^{**1**}**H-NMR** (CO(CD₃)₂): δ in ppm = 1.21 (t, 3H, ^{*3*}J = 7.21 Hz; CH₂C*H*₃), 1.32 - 2.73 (m, 20H; 10 CH₂), 2.63 (q, 2H, ³*J* = 7.17 Hz; CH₂CH₃), 3.61 (s, 3H; OCH₃), 6.25 (d, 1H, ³*J* = 3.60 Hz; CH_{*arom*}), 6.51 (d, 1H, ³*J* = 5.24 Hz; C*H*_{*alken*}), 6.77 (d, 1H, ³*J* = 3.54 Hz; CHₐᵣₒₘ), 7.31 (d, 1H, ³*J* = 12.97 Hz; CHₐₗₖₑₙ). ^{**13**}**C-NMR** (CO(CD₃)₂): δ in ppm = 14.58 (*C*H₃), 25.67 - 34.65 (11*C*H₂), 51.41 (O*C*H₃), 110.29 (*C*Hₐᵣₒₘ), 118.42 (*C*Hₐᵣₒₘ), 123.36 (*C*Hₐₗₖₑₙ), 128.63 (*C*Hₐₗₖₑₙ), 151.89 *(CC*_{*arom*}*),* 162.76 (C*C*_{*arom*}), 174.11 (COOCH₃), 199.28 (CO). MS (EI, 111 °C), m/z (%) = 348 (35.11) [M⁺], 346 (36.69) [M⁺-H₂], 319 (19.70) [M⁺- C₂H₅], 291 (7.42) [M⁺-C₄H₉], 261 (17.99) [M⁺- C₄H₇O₂], 163 (15.51) [M⁺- C₁₁H₂₁O₂], 149 (100.0) [C₉H₉O₂⁺], 109 (60.03) [C₇H₉O⁺], 57 (55.92) [C₃H₅O⁺], 55 (50.46) [C₃H₃O⁺]. IR (Film): 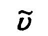 = 3456 (w), 3118 (m), 2928 (s), 2855 (s), 1740 (s), 1685 (s), 1610 (s), 1518 (m), 1464 (m), 1437 (m), 1364 (m), 1255 (m), 1199 (s), 1172 (s), 1119 (s), 1019 (s), 970 (s), 797 (s) cm⁻¹. HRMS (C₂₁H₃₂O₄)[m/z]: 348.2300 (ber. 348.2302).

### Beispiel 13

### 11-[5(3-Oxo-butyl)-furan-2-yl]-undecansäure-methylester (13a)

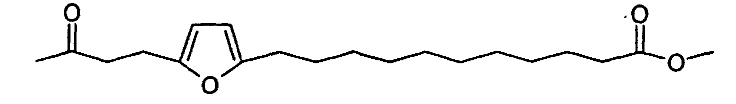

Eine Suspension von 406 mg **11a** (1.23 mmol) und 100 mg Ruthenium auf Aluminium in 20 ml Methanol wurde für 36 Stunden bei 1 atm H₂-Überdruck hydriert. Der Katalysator wurde abfiltiert und das Lösungsmittel abdestilliert. Nach säulenchromatographischer Reinigung (Aluminiumoxid // n-Hexan : Essigester = 3:2) wurde **13a** in Form eines braunen Öls erhalten (Ausbeute: 282 mg = 68%, Rᵣ-Wert = 0.89).

^{**1**}**H-NMR** (CO(CD₃)₂):δ in ppm = 0.89 - 2.83 (m, 27H; 12 CH₂, 1 CH₃), 3.61 (s, 3H; OCH₃), 5.85 (d, 1H, ³*J* = 3.44 Hz; C*H*_{*arom*}), 5.88 (d, 1H, ³*J* = 3.46 Hz; C*H*_{*arom*}). ^{**13**}**C-NMR** (CO(CD₃)₂):δ in ppm = 23.21 - 41.86 (12*C*H₂), 29.83 (H₃*C*CO), 51.43 (O*C*H₃), 105.79 (*C*Hₐᵣₒₘ), 106.14 (*C*Hₐᵣₒₘ), 153.83 (C*C*_{*arom*}), 155.40 (CCₐᵣₒₘ), 174.10 (*C*OOCH₃), 206.81 (CO). **MS** (EI, 95°C), m/z (%) = 336 (21.99) [M⁺], 278 (18.28) [C₁₈H₂₃O₂⁺], 137 (6.49) [C₈H₅O₂⁺], 135 (49.32) [C₈H₇O₂⁺], 109 (20.42) [C₇H₈O⁺], 107 (24.97) [C₇H₇O⁺], 95 (52.25) [C₆H₇O⁺], 57 (35.74) [C₃H₅O⁺], 55 (57.32) [C₃H₃O⁺], 43 (100.0) [C₂H₃O⁺]. IR (Film): 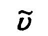 = 3447 (m), 3101 (m), 2926 (s), 2855 (s), 1740 (s), 1718 (s), 1559 (m), 1457 (m), 1437 (m), 1363 (m), 1257 (m), 1197 (m), 1170 (m), 1071 (s), 1012 (m), 783 (m) cm⁻¹. **HRMS** (C₂₀H₃₂O₄)[m/z]: 336.2300 (ber. 336.2298).

### Beispiel 14

### 11-[5(3-Oxo-pentyl)-furan-2-yl]-undecansäure-methylester(13b)

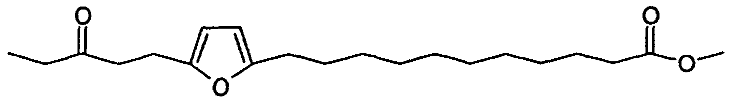

Eine Suspension von 420 mg 11b (1.23 mmol) und 100 mg Ruthenium auf Aluminium in 20 ml Methanol wurde für 36 Stunden bei 1 atm H₂-Überdruck hydriert. Der Katalysator wurde abfiltiert und die Lösung eingeengt. Nach säulenchromatographischer Reinigung (Aluminiumoxid // n-Hexan : Essigester = 3:2) wurde **13b** in Form eines braunen Öls erhalten (Ausbeute: 306 mg = 71%; R_{f}-Wert = 0.82).

^{**1**}**H-NMR** (CO(CD₃)₂): δ in ppm = 0.98 (t, 3H, ³*J* = 7.21 Hz; CH₂CH₃), 1.23 - 2.84 (m, 24H; 12 CH₂), 3.59 (s, 3H; OCH₃), 4.45 (q, 2H, ³*J* = 7.12 Hz; CH₂CH₃), 5.87 (d,1 H, ³J = 3.11 Hz; *CH*_{*arom*}*),* 5.88 (d, 1H, ³*J* = 4.16 Hz; C*H*_{*arom*}). ^{**13**}**C-NMR**(CO(CD₃)₂): δ in ppm = 7.98 (*C*H₃), 23.64 - 40.78 (13*C*H₂), 51.41 (O*C*H₃), 106.07 (*C*Hₐᵣₒₘ), 106.18 (*C*Hₐᵣₒₘ), 153.94 (CC_{*arom*} ), 155.44 ((C*C*_{*arom*}*),* 174.09 (COOCH₃), 208.92 (CO). MS (EI, 114°C), m/z (%) = 350 (32.56) [M⁺], 319 (13.00) [M⁺- OCH₃], 293 (10.33) [M⁺-C₃H₅O], 279 (13.54) [M⁺-C₄H₇O]. 278 (23.86) [M⁺-C₄H₈O], 165 (73.12) [C₁₀H₁₃O₂⁺], 149 (36.27) [C₉H₉O₂⁺], 135 (16.91) (C₉H₇O₂⁺], 121 (19.70) [C₇H₅O₂⁺], 109 (22.17) [C₇H₉O⁺], 57 (100.0) [C₃H₅O⁺], 55 (62.07) [C₃H₃O⁺], 43 (37.68) [C₂H₃O⁺].IR (Film): 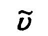 = 3103 (m), 2927 (s), 2854 (s), 1739 (s), 1720 (s), 1568 (m), 1462 (m), 1436 (m), 1368 (m), 1254 (w), 1172 (m), 1113 (w), 1013 (m), 781 (m) cm⁻¹. **HRMS** (C₂₁H₃₄O₄)[m/z]: 350.2457 (ber. 350.2459).

### Beispiel 16

### Wirksamkeitsnachweis der Verbindungen 11a/11b, 12b, 6b, 13b und 14 (mit n = 8)

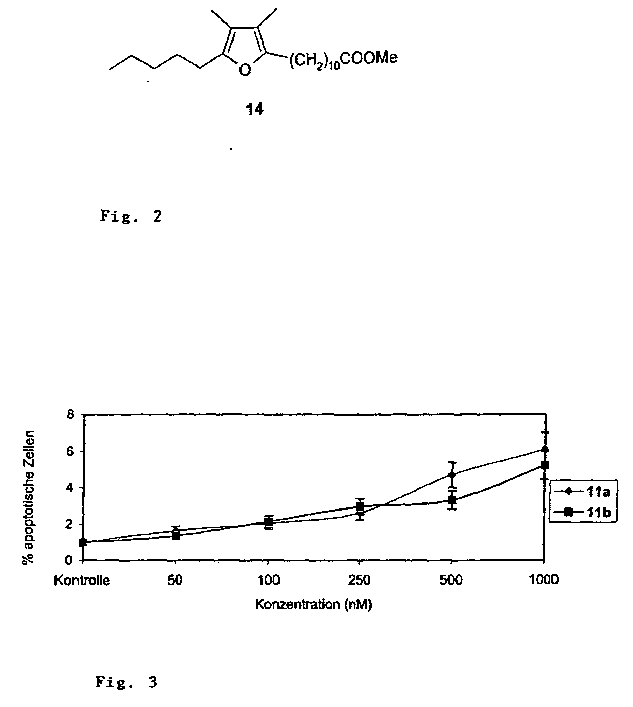

Untersuchungen der pro- bzw. antiapoptotischen Wirkung der FFS wurden an einer Nierenkarzinomzellinie (RENCA; Murphy et al., J. Natl. Cancer Inst. 50: 1013, 1973) durchgeführt. Dazu wurden die Zellen 22 Stunden zum einen mit einem Zytostatikum, zum anderen mit der entsprechenden FFS sowie einer Kontrolle und einer Kombination aus FFS und Zytostatikum inkubiert. Für die Substanzkombination aus FFS und Zytostatikum wurden die Zellen zwei Stunden mit der jeweiligen FFS vorinkubiert, anschließend das Zytostatikum zugegeben und danach für 24 Stunden im Brutschrank inkubiert. Die verwendeten Konzentrationen sind in Figur 3 angegeben. Diese Untersuchungen wurden mit den **Verbindungen 11a/11b, 12b, 6b, 13b und 14** sowie jeweils für jedes Zytostatikum getrennt durchgeführt. Es zeigte sich bei keiner der getesteten FFS (alleinige Zugabe) eine signifikante Zunahme der Zahl apoptotischer Zellen gegenüber der Kontrolle. Bei Inkubation der Zellen mit einer FFS und anschließender Zytostatikumgabe konnten unterschiedliche Effekte beobachtet werden .

Bei den alken- und alkansubstituierten FFS **12b, 6b, 13b, 14** war bei den beiden Zytostatika (Daunorubicin und Vinblastin; Fa. Rhone-Poulenc Rorer) ein leichter Rückgang der Anzahl apoptotischer Zellen zu beobachten, unabhängig von der Wahl des Zytostatikums. Bei den alkin-substituierten FFS wurde bei Verwendung von Daunorubicin in Kombination mit **11a** eine Zunahme von 60% und in Kombination mit **11b** eine Abnahme der Anzahl apoptotischer Zellen gemessen. Bei Verwendung von Vinblastin als Zytostatikum nahm bei beiden FFS **11a** und **11b** die Zahl apoptotischer Zellen von 60% bzw. 160% zu.

Aufgrund dieser Effekte wurde mit den alkin-substituierten FFS **11a** und **11b** an einer weiteren Karzinomzelllinie (Morris-Hepatom; Tumorbank DKFZ Heidelberg, TZB No. 620143, MH TC 5123, histologischer Typ: Hepatom, Herkunft/Spezies: Ratte, Buffalo) die oben beschriebenen Untersuchungen durchgeführt. Die Konzentrationen der verwendeten FFS bzw. Zytostatika sind in Figur 4 angegeben. FFS alleine zeigten auch hier keine Effekte. Bei einer Kombination aus Daunorubicin mit der FFS **11a** bzw. **11b** stieg die Zahl apoptotischer Zellen um 130% bzw. 60% und bei einer Kombination FFS 11 a bzw. 11 b mit Vinblastin um 85% bzw. 50%.

Da die FFS **11a** und **11b** als einzige aller synthetisierten FFS eine signifikante Zunahme an apoptotischen Zellen bei Inkubation in Kombination mit einem Zytostatikum zeigten, wurde untersucht, ob dieser Effekt von der Konzentration der FFS abhängt. Daher wurde das oben beschriebene Experiment an Renca-Zellen unter den gleichen Bedingungen, aber mit jeweils verschiedenen Konzentrationen von **11a** bzw. **11b** im Bereich von 0 bis 1000 nM durchgeführt (Figur 3). Dabei wurde eine deutliche Konzentrationsabhängigkeit festgestellt. Der prozentuale Anteil apoptotischer Zellen nahm mit steigender FFS Konzentration zu und mit fallender ab. Bei Inkubation mit einer 1000 nM-Lösung wurde eine 6.1 bzw. 5.2-fach höhere Anzahl apoptotischer Zellen im Vergleich zur Kontrolle mit Daunorubicin alleine festgestellt. Bei Verwendung einer 50 nM-Lösung war die Zahl apoptotischer Zellen ca. halb so groß wie bei Verwendung einer 100 nM-Lösung. Bei Inkubation der Zellen mit FFS ohne Zytostatikum wurden auch bei hoher FFS Konzentration keine signifikante Zu- bzw. Abnahme der Apoptoserate festgestellt. Die Apoptoserate wurde routinemäßig durch Messung YO-PRO-1 positiver Zellen^{5,6} DNA-Leiter-Untersuchungen ("DNA-laddering") ermittelt.

### Literaturverzeichnis

[1] a) Y. Kojima, S. Wakita, N. Kato, *Tetrahedron Lett.,* **1979,** *47*, 4577; b) B. H. Lipshutz, R. S. Wilhelm, J. A. Kozlowski, *J. Org. Chem.,* **1984,** *49*, 3938.
[2] B. L. Feringa, R. Hulst, R. Rikers, L. Brandsma, *Synthesis,* **1988,** 316.
[3] P. F. Schuda, C. B. Ebner, S. J. Potlock, *Synthesis,* **1987,** 1055.
[4] a) J. C. Stowell, D. R. Keith, *Synthesis,* **1979,** 132; b) Y. Le Bigot, M. Delmas, A. Gaset, *Tetrahedron,* **1988,** *44,* 1057.
[5] Idziorek, T., Estaquier, J., De Bels, F., und Ameisen, J. C. (1995) YOPRO-1 permits cytofluorometric analysis of programmed cell death (apoptosis) without interfering with cell viability. *J. Immunol. Methods* **1995,** *185,* 249
[6] Deigner, H.P., Claus, R., Bonaterra, G.A., Gehrke, C., Bibak, N., Blaess, M., Cantz, M., Metz, J., Kinscherf, R. Ceramide induces aSMase expression: Implications for oxLDL-induced apoptosis. *FASEB J. ,* im Druck

## Patentansprüche

1. Verbindung mit der Formel 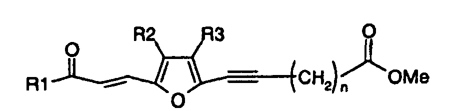 wobei
R1, R2 und R3, die gleich oder verschieden sein können, folgende Bedeutungen haben:
Wasserstoff,
Hydroxyl,
C₁-C₂₀ Alkyl-, Alkenyl-, Alkinyl-, Alkylthioreste O-Alkyl
COOH-, CONH₂-, CONH-Alkyl-, CON(Alkyl)₂ - Rest (mit Alkyl = C₁ - C₁₀)
Nitro,
Amino, Alkylaminorest (mit Alkyl = C₁ - C₁₀),
Aryl-, Aralkyl-, Alkoxyarylrest(wobei Aryl einen mono- oder polycyclischen carbo- oder heterocyclischen Rest bedeutet),
sowie R2 und/oder R3:
Halogen,
und
n eine ganze Zahl im Wert von 1 bis 20 ist,
oder
ein pharmazeutisch verträgliches Salz davon.

2. Verbindung nach Anspruch 1, wobei R1 ein Methyl-, Ethyl- oder Propylrest ist und R2 und R3 ein Methylrest oder Wasserstoffatom sind

3. Verbindung nach Anspruch 2, wobei R1 ein Methylrest oder Ethylrest ist und R2 und R3 jeweils Wasserstoffatome sind.

4. Verbindung nach Anspruch 3, wobei n 8 ist.

5. Verbindung nach Anspruch 4, die 11-[5-(3-Oxo-1-butenyl)-furan-2-yl]-10-undecinsäure-methylester **(11a)** oder 11-[5-(3-Oxo-1-pentenyl)-furan-2-yl]-10-undecinsäure-methylester **(11b)** ist.

6. Arzneimittel, umfassend eine pharmakologisch wirksame Menge einer Verbindung nach einem der Ansprüche 1 bis 5 zusammen mit einem pharmazeutisch verträglichen Träger.

7. Verwendung einer Verbindung nach einem der Ansprüche 1 bis 5 zur Herstellung eines Arzneimittels zur Steigerung der Wirksamkeit einer Zytostatikatherapie und/oder Bestrahlungstherapie.

8. Verwendung einer Verbindung nach einem der Ansprüche 1 bis 5 zur Herstellung eines Arzneimittels zur Verhinderung oder Reduktion einer Resistenzbildung bei einer Zytostatikatherapie und/oder Bestrahlungstherapie.

9. Verwendung nach Anspruch 7 oder 8, wobei das in der Zytostatikatherapie verwendete Zytostatikum Daunorubicin oder Vinblastin ist.

## Claims

1. Compound of the formula 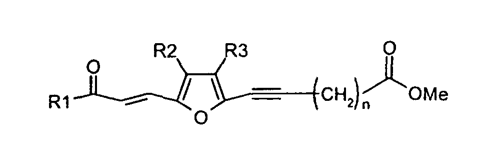 in which
R1, R2 and R3 which may be equal or different, represent
a hydrogen atom
a hydroxyl group
a C₁ - C₂₀ alkyl, O-alkyl, alkenyl, alkinyl, thioalkyl residue
a C₁ - C₁₀ alkoxy residue
a COOH, CONH₂, CONH-alkyl, CON(alkyl)₂ residue (whereas alkyl = C₁ - C₁₀)
a nitro group,
an amino, alkylamino residue (whereas alkyl = C₁ - C₁₀),
an aryl, aralkyl, alkoxyaryl residue,
a halogen atom,
and
n is an even number from 1 to 20, and
whereas the compound is further **characterized in that** it can improve the efficiency of a cytostatic and/or radiation therapy,
or
a pharmaceutically compatible salt thereof.

2. Compound according to claim 1, whereas R1 is a methyl, ethyl or propyl residue and R2 and R3 are a methyl residue or a hydrogen atom.

3. Compound according to claim 2, whereas R1 is a methyl residue or a ethyl residue and R2 and R3 are a hydrogen atom each.

4. Compound according to claim 3, whereas n is 8.

5. Compound according to claim 4, which is 11-[5-(3-oxo-1-butenyl)-furan-2-yl]-10-undecinacid-methylester (11a) or 11-[5-(3-oxo-1-pentenyl)-furan-2-yl]-10-undecinacid-methylester (11b).

6. Pharmaceutical preparation comprising a pharmaceutical effective amount of a compound according to anyone of the claims 1 to 5 together with a pharmaceutical compatible carrier.

7. Use of a compound according to anyone of the claims 1 to 5 for improving the efficiency of a cytostatic and/or radiation therapy.

8. Use of a compound according to anyone of the claims 1 to 5 for preventing or reducing the formation of resistances by a cytostatic and/or radiation therapy.

9. Use according to claim 7 or 8, whereas the cytostatic agent used in the cytostatic therapy is daunorubicin or vinblastine.

## Revendications

1. Composé de formule 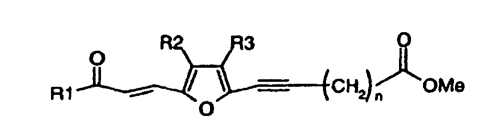 dans laquelle
R1, R2 et R3, qui peuvent être identiques ou différents, ont les définitions suivantes :
hydrogène,
hydroxyle,
reste alkyle, alcényle, alcynyle, alkylthio, O-alkyle en C₁ à C₂₀,
reste COOH, CONH₂, CONH-alkyle, CON-(alkyle)₂, (avec alkyle en C₁ à C₁₀),
nitro,
amino, reste alkylamino (avec alkyle en C₁ à C₁₀),
reste aryle, aralkyle, alkoxyaryle (où aryle désigne un reste monocyclique ou polycyclique, carbocyclique ou hétérocyclique),
ainsi que R2 et/ou R3:
halogène,
et
n est un nombre entier de valeur allant de 1 à 20,
ou
un sel acceptable du point de vue pharmaceutique de ce composé.

2. Composé suivant la revendication 1, dans lequel R1 est un reste méthyle, éthyle ou propyle et R2 et R3 représentent un reste méthyle ou un atome d'hydrogène.

3. Composé suivant la revendication 2, dans lequel R1 est un reste méthyle ou éthyle et R2 et R3 représentent chacun un atome d'hydrogène.

4. Composé suivant la revendication 3, dans lequel n est égal à 8.

5. Composé suivant la revendication 4, qui est l'ester méthylique d'acide 11-[5-(3-oxo-1-butényl)-furanne-2-yl]-10-undécynoïque (11a) ou l'ester méthylique d'acide 11-[5-(3-oxo-1-pentényl)-furanne-2-yl]-10-undécynoïque (11b).

6. Médicament comprenant une quantité pharmacologiquement efficace d'un composé suivant l'une des revendications 1 à 5 conjointement avec un support acceptable du point de vue pharmaceutique.

7. Utilisation d'un composé suivant l'une des revendications 1 à 5 pour la préparation d'un médicament destiné à accroître l'activité d'une thérapie par cytostatique et/ou d'une radiothérapie.

8. Utilisation d'un composé suivant l'une des revendications 1 à 5 pour la préparation d'un médicament destiné à empêcher ou à réduire le développement d'une résistance dans une thérapie par cytostatique et/ou une radiothérapie.

9. Utilisation suivant la revendication 7 ou 8, dans laquelle le cytostatique utilisé dans la thérapie par cytostatique est la daunorubicine ou la vinblastine.
